# EUROPEAN PATENT APPLICATION

(11) **EP 3 666 280 A1**
(43) Date of publication of application: **17.06.2020**
(21) Application number: 19215669.3
(22) Date of filing: 12.12.2019
(51) Int. Cl.: A61K 36/185

(54) **METHOD FOR THE EXTRACTION OF ACTIVE PRINCIPLES FROM CANNABIS**

(30) Priority: 14.12.2018 IT 201800011128
(71) Applicant: Università Degli Studi Di Torino, 10124 Torino (IT)
(72) Inventor: BRUSA, Paola, I-10146 Torino (IT); BARATTA, Francesca, I-14048 Montegrosso d'Asti (Asti) (IT)
(74) Representative: Freyria Fava, Cristina

(57) **Abstract**

A method for extracting delta-9-tetrahydrocannabinol (THC) and cannabidiol (CBD) from *Cannabis* inflorescences, wherein the method comprises the following steps:
(i) triturating the inflorescences of *Cannabis* obtaining a triturated mass;
(ii) preheating the triturated mass in an oven at a temperature between 120 and 145°C, wherein the triturated mass subjected to preheating has a thickness of less than or equal to 5 mm, obtaining a preheated triturated mass;
(iii) mixing the preheated triturated mass with a lipophilic solvent, obtaining a solid-liquid mixture;
(iv) heating the solid-liquid mixture in a water bath with boiling water;
(v) separating a lipophilic liquid phase and a solid phase from the heated solid-liquid mixture,
where the lipophilic liquid phase contains THC and CBD.

## Description

### Field of the invention

The present description relates to a method for extracting active principles from *Cannabis.*

### Background of the invention

Recently, Italian legislation has considered the possibility of the regulated medical use of *Cannabis* in the field of pain therapy, to counteract nausea and vomiting caused by chemotherapy and radiotherapy, as an appetite stimulant in cachexia, anorexia, in inappetent patients with cancer or with AIDS and in a couple of additional pathological conditions, i.e. glaucoma and Gilles de la Tourette syndrome.

In the phytocomplex of *Cannabis* there are over 500 different molecules of which around one hundred belong to the chemical class of cannabinoids. The molecules of greatest medical interest are delta-9-tetrahydrocannabinol ("THC") and cannabidiol ("CBD") in the decarboxylated forms.

To date, two cannabinoid receptor isoforms have been identified in the human body: CB1 type receptors and CB2 type receptors. CB1 type receptors are ubiquitously present in the brain, where they have high concentrations especially in some areas that are related to the many clinical actions of *Cannabis.* CB2 type receptors are only partially present in the brain, but are more common in the periphery, where they demonstrate an action on immune function and inflammation.

THC acts mainly through CB1 type receptors and CBD exerts some action on CB2 type receptors. Some relevant clinical consequences derive from this knowledge: the action on pain is more powerful when combining THC and CBD, rather than through administering THC alone, and the presence of CBD reduces the psychomimetic effects of THC, reducing the risk of abuse. It follows that it is very important to know the qualitative-quantitative content of at least the two main cannabinoids mentioned above present in the formulations that are administered to the patient.

The only medicinal product of industrial origin based on *Cannabis* which has currently obtained marketing authorization is Sativex spray for treating spasticity due to multiple sclerosis. Each spray dose contains 2.7 mg of THC and 2.5 mg of CBD, with a maximum daily dose of 32.4 mg of THC and 30 mg of CBD.

The current legislation in Italy envisages that administering *Cannabis* for medical use can take place orally or by inhalation. Regarding galenic preparations, relative to the oral route of administration, the pharmaceutical form to be considered as the first choice is decoction, but administration in the form of oil is also envisaged. The inhalation route is to be considered if oral administration does not produce the required pharmacological effects or when the attending physician deems it appropriate.

With reference to decoction, it has been observed that acid cannabinoids are more present than the corresponding decarboxylated cannabinoids, consequently with the decoction, patients do not take a quantity of THC and CBD that is useful for medical purposes, as a maximum of 500 ml of decoction/day is usually taken. A quantitative analysis of the THC and CBD content for decoctions prepared according to the indications provided by the Ministry of Health (100 mg of Cannabis per 100 ml of water) has, in fact, provided the data shown in Table 1 and allowed calculation of a ratio between the acid and decarboxylated fractions equal to 2.30±0.218 for THC and 4.85±0.315 for CBD.

**Table 1**

| **Method** | **Active molecule** | **Number of analyzed samples** | **Average of the active molecule content mg/100 ml** | **S.D.** | **Active molecule content: minimum value mg/100 ml** | **Active molecule content: maximum value mg/100 ml** |
|---|---|---|---|---|---|---|
| **A-1 (100 mg of *Cannabis* In 100 ml of water)** | THC | 39 | 0.87 | 0.41 | 0.26 | 2.47 |
| | CBD | | 1.07 | 0.43 | 0.27 | 2.42 |
| | THCA | | 1.81 | 0.55 | 0.88 | 3.09 |
| | CBDA | | 4.83 | 1.07 | 2.59 | 7.71 |
| | CBN¹ | | 0.02 | 0.03 | 0.00 | 0.15 |
| **A-2 (200 mg of *Cannabis* in 200 ml of water)** | THC | 6 | 0.72 | 0.15 | 0.53 | 0.88 |
| | CBD | | 1.01 | 0.14 | 0.86 | 1.24 |
| | THCA | | 1.81 | 0.89 | 0.58 | 2.52 |
| | CBDA | | 5.18 | 0.91 | 3.87 | 6.38 |
| | CBN | | N.D. | N.D. | N.D. | N.D. |
| **A-3 (300 mg of *Cannabis* in 300 ml of water)** | THC | 6 | 0.83 | 0.26 | 0.59 | 1.31 |
| | CBD | | 1.09 | 0.26 | 0.90 | 1.62 |
| | THCA | | 1.91 | 0.78 | 0.92 | 2.60 |
| | CBDA | | 5.36 | 0.63 | 4.29 | 6.12 |
| | CBN | | N.D. | N.D. | N.D. | N.D. |

| | | | | | | |
|---|---|---|---|---|---|---|
| ¹ Cannabinol (CBN) | | | | | | |

Regarding the oily preparations for oral use, there are substantially three methods currently known and used for preparing galenic preparations based on *Cannabis:* the one called "Romano-Hazekamp", the one called "Citti-Cannazza" and the one called "SIFAP".

The first two methods envisage that *Cannabis* is previously chopped and then mixed with olive oil. The resulting mixture is then heated (for 2 hours in a water bath with boiling water for the "Romano-Hazekamp" method, and for 2 hours at 110°C for the "Citti-Cannazza" method) and then filtered to obtain the oily extract.

The "SIFAP" method instead envisages that the *Cannabis* is chopped and then preheated to 115°C for 40 minutes. For preheating, the drug is distributed in a 1 cm-height layer. Subsequently the *Cannabis* is transferred to the olive oil and further crushed with a turbomulsifier for 3 minutes. The mixture of *Cannabis* and olive oil is then heated in a water bath with boiling water for 40 minutes, filtered and supplemented with 0.02% butylhydroxyituoluene (BHT).

All the methods in question envisage that the weight (g)/volume (ml) ratio between drug and solvent is equal to 0.1:1.

Based on what is reported in the literature, if we analyze the quantities of active molecules that can be obtained by applying the three above methods, we obtain the following results for the variety of *Cannabis* FM2 (Table 2):

**Table 2**

| **METHOD** | **% THC** | **% CBD** | **% THCA** | **% CBDA** |
|---|---|---|---|---|
| Romano-Hazekamp | 0.17 ± 0.09 | 0.05 ± 0.03 | 0.18 ± 0.06 | 0.47 ± 0.10 |
| Citti-Cannazza | 0.24 ± 0.08 | 0.23 ± 0.11 | 0.24 ± 0.14 | 0.67 ± 0.25 |
| SIFAP | 0.37 ± 0.08 | 0.70 ± 0.19 | 0.07 ± 0.07 | 0.15 ± 0.09 |

The most effective method, known as "SIFAP", allows at most to obtain an oil whose THC concentration is equal to 0.37% ± 0.08 (3.38 mg/ml) and the CBD concentration is equal to 0.70% ± 0.19 (6.40 mg/ml).

The known methods for extracting THC and CBD from *Cannabis* in the form of oil therefore do not allow a significant extraction of the aforesaid active principles.

### Summary of the invention

Taking these premises into consideration, the need is therefore felt to develop a method for extracting THC and CBD from *Cannabis* which overcomes the aforesaid disadvantages, allowing an industrial production of these active principles for their respective use in medicine.

In accordance with the invention, the aforesaid object is achieved thanks to the solution specifically recalled in the attached claims, which form an integral part of the present description.

One embodiment of the present invention relates to a method for extracting delta-9-tetrahydrocannabinol (THC) and cannabidiol (CBD) from *Cannabis* inflorescences, wherein the method comprises the following steps:
(i) triturating the inflorescences of *Cannabis* obtaining a triturated mass;
(ii) preheating the triturated mass in an oven at a temperature between 120 and 145°C, wherein the triturated mass subjected to preheating has a thickness of less than or equal to 5 mm, obtaining a preheated triturated mass;
(iii) mixing the preheated triturated mass with a lipophilic solvent, obtaining a solid-liquid mixture;
(iv) heating the solid-liquid mixture in a water bath with boiling water;
(v) separating a lipophilic liquid phase and a solid phase from the heated solid-liquid mixture,
where the lipophilic liquid phase contains THC and CBD.

The present description also relates to a solid formulation containing THC and CBD, wherein the solid formulation comprises a solid matrix and a lipophilic liquid phase containing THC and CBD, wherein the lipophilic liquid phase containing THC and CBD is obtained by carrying out the aforesaid method.

### Detailed description of the invention

In the following description, there are numerous specific details to provide a thorough understanding of the embodiments. The embodiments may be implemented in practice without one or more of the specific details, or with other methods, components, materials, etc. In other cases, well-known structures, materials or operations are not shown or described in detail to avoid obscuring certain aspects of the embodiments.

Throughout the present specification, the reference to "an embodiment" or "embodiment" means that a particular configuration, structure, or characteristic described in connection with the embodiment is included in at least one embodiment. Therefore, the appearance of expressions "in a certain embodiment" or "in an embodiment" in various point throughout this specification does not necessarily always refer to the same embodiment. Moreover, the particular details, structures or characteristics can be combined in any suitable way in one or more embodiments.

The headings used here are used merely for convenience and do not interpret the object or meaning of the embodiments.

The present description concerns a method for extracting the active principles delta-9-tetrahydrocannabinol (THC) and cannabidiol (CBD) in a decarboxylated form from *Cannabis* inflorescences, obtaining an almost quantitative extraction of these active principles at the end of the method.

In one embodiment, the method subject of the present description comprises the following steps:
(i) triturating the inflorescences of *Cannabis* obtaining a triturated mass;
(ii) preheating the triturated mass in an oven at a temperature between 120 and 145°C, wherein the triturated mass subjected to preheating has a thickness of less than or equal to 5 mm, obtaining a preheated triturated mass;
(iii) mixing the preheated triturated mass with a lipophilic solvent, obtaining a solid-liquid mixture;
(iv) heating the solid-liquid mixture in a water bath with boiling water;
(v) separating a lipophilic liquid phase and a solid phase from the heated solid-liquid mixture,
where the lipophilic liquid phase contains THC and CBD.

In one embodiment, the lipophilic solvent is selected from virgin olive oil and a pharmaceutically acceptable vegetable oil, preferably virgin olive oil.

In one embodiment, the mixing step (iii) of the triturated mass preheated with a lipophilic solvent is carried out in a weight (g)/volume (ml) ratio within the range from 0.1:1 to 0.3:1, preferably equal to 0.2:1.

In one embodiment, separating of the phase (v) envisages subjecting the heated solid-liquid mixture to a pressing operation and/or filtering the heated solid-liquid mixture with a filter having a pore diameter between 0.45 micrometers and 3 millimeters.

In one embodiment, the heating step (iv) is carried out for a period of time from 30 to 120 minutes, preferably equal to 60 minutes.

In one embodiment, the preheating step (ii) is carried out at a temperature of 140°C for a period of time from 20 to 45 minutes, preferably 30 minutes.

In one embodiment, at the end of step (iv), the heated solid-liquid mixture is allowed to cool, preferably for a period of time from 10 to 40 minutes.

The present description also relates to a solid formulation containing THC and CBD, wherein the solid formulation comprises a solid matrix and a lipophilic liquid phase containing THC and CBD, wherein the lipophilic liquid phase containing THC and CBD is obtained by carrying out the aforesaid method.

In one embodiment, the solid matrix is made up of excipients for oral use capable of gelling or adsorbing the lipophilic liquid phase containing THC and CBD. In a preferred embodiment, the solid matrix is selected from silica, starch, talc, magnesium stearate or a mixture thereof, preferably silica.

In one embodiment, the solid matrix is present with respect to the lipophilic liquid phase containing THC and CBD in a weight ratio between 1:5 and 1:70, preferably equal to 1:50.

In one embodiment, the solid formulation is encapsulated in a gelatin capsule for pharmaceutical use, or is pressed into a tablet form, optionally coated with a pharmaceutically acceptable coating film.

The lipophilic liquid phases obtained with the method described here have been analyzed in order to determine the amount of THC and CBD both in acid and in decarboxylated form. Based on the results obtained, reproduced in Table 5, it is possible to affirm that the preheating of the plant drug is very useful for obtaining lipophilic liquid phases in which the molecules present are - for the most part - in the decarboxylated form and therefore of pharmacological interest.

As for the conditions applied for preheating, the best results were obtained by applying more intense heating for a shorter time (140°C for 30 minutes) rather than milder heating for a longer time (115°C for 40 minutes).

It has also been observed that the thickness of the drug subjected to preheating plays a fundamental role on the quantity of THC and especially of CBD extracted in decarboxylated form. In particular, it has been observed that by subjecting the drug to oven preheating, which has a thickness of no greater than 5 mm, better results are obtained in terms of THC and especially of CBD extracted in decarboxylated form. Higher drug thicknesses instead lead to a greater presence of molecules in acid form. In particular, it has been experimentally determined that if the thickness of the plant drug is greater than 5 mm, the quantity of molecules in acid form, especially CBD, can increase up to 10 times.

As for the boiling water bath, the results obtained allow us to affirm that its duration does not considerably influence the content of active molecules of the finished product.

When taking into consideration the ratio between drug and lipophilic solvent and the concentration of active molecules in the lipophilic liquid phase obtained at the end of the method described here, it is possible to affirm that, doubling the quantity of drug used, with other conditions being equal, the concentration of THC and CBD in the lipophilic liquid phase increases by more than double. In fact, the ratio between the THC obtained with the β methods and the THC obtained with the α methods is greater than 2, the same for CBD. Therefore the methods of series β allow a more efficient extraction of THC and CBD.

The extraction method of THC and CBD described herein allows - on average - to obtain 8.04 mg/ml of THC and 13.05 mg/ml of CBD, both in decarboxylated form. These are significantly higher values than those obtainable with extraction in the aqueous phase (decoction) and in the oil phase according to the currently known methods, i.e. "Romano-Hazekamp", "Citti-Cannazza" and "SIFAP". The high content of active molecules in decarboxylated form of the lipophilic liquid phases obtained with the method described in the present description allows both a reduction of the quantities of raw material to be used and consequently of the related purchase costs, and a lower volume of preparation to be administered to the patients to obtain the required dosage.

Concerning the evaluation of the stability of the prepared preparations, it can be underlined that, for lipophilic preparations, storage in the refrigerator is not necessary at least for six months. This entails a considerable facilitation for patients in managing the prescribed therapy.

Furthermore, since the encapsulation of the lipophilic liquid phase does not affect its stability, this pharmaceutical form represents the one of choice for administration to patients both because the capsule masks the unpleasant organoleptic characteristics of the preparation, and because it makes the formulation more easily manipulable and transportable, both at the industrial preparation level and by patients.

### Materials and methods

### Materials for preparing galenic preparations

All the galenic preparations described have been set up with FM2 type *Cannabis* inflorescences intended for technological research use, purchased at the Military Pharmaceutical Chemical Plant in Florence, whose concentration of active molecules was, before the experiments were carried out (May 2017), equal to 0.40±0.02% for THC, 5.74%±0.18% for THCA, 0.29%±0.03% for CBD, 8.70±0.17% for CBDA. Consequently, the total THC, calculated with the formula %THC tot = %THC + (0.877 x %THCA), was equal to 5.43%±0.15% and the total CBD, calculated with the formula %CBD tot = %CBD + (0.877 x %CBDA), was 7.92%10.18%. At the end of the experiments (June 2018) the concentration of the plant drug, calculated in the same way, was re-evaluated and was found to be 2.54%±0.33% for THC, 2.97%±0.41% for THCA, 1.71%±0.26% for CBD, and 6.29%±0.72% for CBDA. Consequently the total THC was equal to 5.14%±0.69% and the total CBD was equal to 7.23%±0.95.

All the other raw materials used for preparing the galenic preparations described below were purchased from a retailer of pharmaceutical raw materials (Farmalabor s.r.l., Canosa di Puglia, Bari, Italy).

Before using *Cannabis* for preparing the formulations described below, the herbal drug was ground in order to make it homogeneous.

### Oil preparation

For preparing *Cannabis* oil, a defined amount of FM2 was placed in a determined volume of virgin olive oil in a weight (g)/volume (ml) ratio of 0.1:1 or 0.2:1. The oil containing the inflorescences was therefore placed under stirring in a boiling water bath for times equal to 30, 60 or 120 minutes. The oil was then filtered with the aid of a press using cotton or cotton wool gauze so as to be able to effectively separate it from the inflorescences of *Cannabis,* obtaining a liquid product.

Before preparing the oil, the inflorescences were arranged in a thin state (maximum 5 mm, preferably between 1 and 2 mm) and placed in an oven for 30 minutes at 140°C, 40 minutes at 115°C or they did not undergo any heat treatment. The volume of the batches prepared was between 5 and 100 ml. Table 3 summarizes the operating conditions applied during preparation of the oils.

**Table 3**

| | **Ratio Solvent/ *Cannabis* (g/ml)** | **Heating time in boiling water bath** | **Preheating temperature of the inflorescences** | **Preheating temperature** |
|---|---|---|---|---|
| α-1 | 0.1:1 | 120 minutes | no preheating | / |
| α-2 | 0.1:1 | 120 minutes | 115°C | 40 minutes |
| α-3 | 0.1:1 | 30 minutes | 140°C | 30 minutes |
| α-4 | 0.1:1 | 60 minutes | 140°C | 30 minutes |
| α-5 | 0.1:1 | 120 minutes | 140°C | 30 minutes |
| β-1 | 0.2:1 | 120 minutes | no preheating | / |
| β-2 | 0.2:1 | 120 minutes | 115°C | 40 minutes |
| β-3 | 0.2:1 | 30 minutes | 140°C | 30 minutes |
| β-4 | 0.2:1 | 60 minutes | 140°C | 30 minutes |
| β-5 | 0.2:1 | 120 minutes | 140°C | 30 minutes |

### Preparation of pharmaceutical forms for oral use

Hard capsules were prepared with the oil prepared using the β-4 method. In particular, after opening the capsules with the help of a manual encapsulator (Farmalabor, Optima Aluminium®), the oil (in variable volumes and between 0.100 and 0.400 ml) was dispensed into the body of the capsules themselves through a pipette (Gilson, Microman®). The remaining space inside the capsules was filled with anhydrous micronized silica (in an amount ranging from 5 mg to 20 mg) and the capsules were then closed. The capsules thus prepared were not removed from the encapsulator which, still with the capsules in place, was overturned by 180 degrees so that the oil slid towards the silica. The capsules were left to rest in this position for 12 hours, after which they were removed from the encapsulator and analyzed to evaluate their uniformity of content according to the indications of the European Pharmacopoeia (document 2.9.6 - EDQM, European Directorate for the Quality of Medicines and HealthCare. European Pharmacopoeia, 9th edition. Geneva: Council of Europe, 2010), as well as stability.

### Analysis methods of the extracted active principles

The chromatographic analysis was carried out using the Acquity® UPLC system coupled with a TQD mass spectrometer (Waters, Milan, Italy). The chromatographic separation was performed with an Acquity UPLC HSS T3 column (2.1 x 30 mm, 1.8 µm; Waters, Milan, Italy) maintained at 30°C. The chromatographic separation was obtained through a gradient of mobile phases A (acetonitrile and water in a ratio of 70:30 + 0.05% of formic acid) and B (isopropanol and acetonitrile in a ratio of 80:20 + 0.05% of formic acid) at 0.4 ml/min. The initial condition of the gradient was represented by 100% of solution A, after 3.5 minutes the mobile phase was brought linearly to 100% of solution B and maintained for 1.5 minutes, then the column was rebalanced to the initial conditions for 1 minute (total time 6.0 minutes). The autosampler was kept at 10°C, the injection volume was 10 µl. Data processing and the system control were managed by MassLynx software (Waters, Milan, Italy). The coupled mass spectrometry was measured in a positive ionization mode (ESI +) with a capillary voltage of 3.50 kV, a temperature source of 150°C and a desolvation temperature of 400°C. The nitrogen flow was 800 L/h and 60 L/h, respectively, for the desolvation and the cone.

Ion monitoring was performed in multiple reaction monitoring mode, with the mass transitions and collision energies (CE) shown below: CBD 315.14 → 193.04, CE 25; CBD-d3 318.10 → 196.14, CE 25; THC 315.11 → 193.05, CE 25; THC-d3 318.19 → 196.12, CE 25; CBDA 359.15 → 219.07, CE 30; THCA 359.13 → 219.11, CEC 30; CBN 311.15 → 223.10, CE 20.

All the standard cannabinoid solutions required for constructing the calibration curve were diluted to concentrations between 1250 ng/ml and 5 ng/ml. CBD-d3 and THC-d3 were used as internal standards.

All the samples to be analyzed were suitably diluted in isopropanol to obtain a final concentration suitable for the interval of the calibration curve.

Pharmaceutical grade olive oil, CBD, CBN, CBDA, cannabidiol-d3 (CBD-d3), THC, tetrahydrocannabinol-d3 (THC-d3) and LC-MS grade isopropanol were purchased from Sigma-Aldrich (Milan, Italy). THCA was purchased from LGC (Milan, Italy). LC-MS grade acetonitrile was purchased from VWR (Milan, Italy). HPLC grade water was produced with the Elix system coupled with the Synergy-UV system for water purification (Merck Millipore, Milan, Italy).

### Stability test

The lipophilic phases obtained at the end of the method described here and the capsules were tested at regular intervals of time following storage in a refrigerator (2-8°C) and at room temperature (15-25°C) in order to evaluate their stability.

The storage conditions of the samples and the timing of the tests are summarized in Table 4. At least three batches were tested for each type of preparation.

**Table 4**

| | | **Days since the date of preparing the preparation** | | | | | | | | |
|---|---|---|---|---|---|---|---|---|---|---|
| | **Storage conditions** | **7** | **15** | **21** | **30** | **60** | **90** | **120** | **150** | **180** |
| **Oils** | Refrigerator | X | X | | X | X | X | X | X | X |
| | Room temperature | X | X | X | X | X | X | X | X | X |
| **Capsules** | Refrigerator | X | X | | X | X | X | X | X | X |
| | Room temperature | | X | | X | X | X | X | X | X |

### RESULTS

### Lipophilic liquid phases

Table 5 shows the quantities of THC, THCA, CBD and CBDA contained in the lipophilic liquid phases obtained at the end of the extraction method described here, and set up with the different operating conditions illustrated in Table 3.

On the basis of the results obtained, it is possible to affirm that only for the methods that do not envisage preheating of the plant drug in the oven (α-1 and β-1), considering the average content of active molecules, the ratio between the acid fraction and the decarboxylated fraction is greater than 1. Regarding the methods that instead envisage preheating, the quantity of active molecules in acid form was greater for the methods that envisage applying a temperature equal to 115°C for 40 minutes (α-2 and β-2) with respect to the methods that envisage applying a temperature of 140°C for 30 minutes (α-3, α-4, α-5, β-3, β-4 and β-5).

Furthermore, on the basis of the results obtained, it can be stated that the duration of the boiling water bath does not tend to influence the content of active molecules of the finished product. In fact, there are no particular differences in the quantity of THC and CBD in the lipophilic liquid phases prepared, respectively, with the methods from α-3 to α-5 for the drug/solvent ratio equal to 0.1:1, and from β-3 to β-5 for the drug/solvent ratio equal to 0.2:1.

Regarding the content of active molecules in the prepared lipophilic liquid phases, it is possible to affirm that, for the same processing conditions applied (time of the boiling water bath, temperature and duration of preheating), the ratio between the THC obtained with the methods β and the THC obtained with the methods α is on average equal to 2.2. Regarding CBD, the ratio in question is on average 2.4.

**Table 5**

| **Method** | **Active molecule** | **Number of samples** | **Average of the active molecule content mg/ml** | **S.D.** | **Active molecule content: minimum value (mg/ml)** | **Active molecule content: maximum value (mg/ml)** |
|---|---|---|---|---|---|---|
| **α-1** | THC | 10 | 0.49 | 0.14 | 0.33 | 0.71 |
| | CBD | | 0.33 | 0.19 | 0.16 | 0.67 |
| | THCA | | 2.67 | 0.45 | 2.07 | 3.29 |
| | CBDA | | 4.40 | 0.84 | 3.12 | 5.45 |
| | CBN | | N.D. | N.D. | N.D. | N.D. |
| **α-2** | THC | 10 | 3.79 | 0.62 | 3.05 | 5.09 |
| | CBD | | 4.26 | 0.69 | 3.32 | 5.45 |
| | THCA | | 0.03 | 0.01 | 0.01 | 0.05 |
| | CBDA | | 1.17 | 0.22 | 0.82 | 1.43 |
| | CBN | | N.D. | N.D. | N.D. | N.D. |
| **α-3** | THC | 6 | 3.35 | 0.22 | 3.07 | 3.65 |
| | CBD | | 5.22 | 0.40 | 4.87 | 5.75 |
| | THCA | | N.D. | N.D. | N.D. | N.D. |
| | CBDA | | 0.09 | 0.03 | 0.04 | 0.13 |
| | CBN | | 0.09 | 0.04 | 0.04 | 0.15 |
| **α-4** | THC | 6 | 3.92 | 0.14 | 3.67 | 4.09 |
| | CBD | | 5.44 | 0.34 | 4.92 | 5.94 |
| | THCA | | N.D. | N.D. | N.D. | N.D. |
| | CBDA | | 0.14 | 0.03 | 0.10 | 0.17 |
| | CBN | | 0.11 | 0.01 | 0.10 | 0.13 |
| **α-5** | THC | 12 | 3.22 | 0.47 | 2.44 | 3.88 |
| | CBD | | 4.98 | 0.67 | 4.07 | 6.58 |
| | THCA | | N.D. | N.D. | N.D. | N.D. |
| | CBDA | | 0.05 | 0.02 | 0.01 | 0.08 |
| | CBN | | 0.08 | 0.02 | 0.04 | 0.11 |
| **β-1** | THC | 4 | 1.94 | 0.11 | 1.82 | 2.09 |
| | CBD | | 1.40 | 0.16 | 1.18 | 1.55 |
| | THCA | | 6.76 | 0.51 | 6.15 | 7.40 |
| | CBDA | | 11.67 | 0.46 | 11.21 | 12.22 |
| | CBN | | N.D. | N.D. | N.D. | N.D. |
| **β-2** | THC | 6 | 7.52 | 0.83 | 6.47 | 8.68 |
| | CBD | | 9.33 | 1.05 | 8.25 | 11.12 |
| | THCA | | 0.11 | 0.04 | 0.07 | 0.17 |
| | CBDA | | 2.85 | 0.44 | 2.36 | 3.45 |
| | CBN | | N.D. | N.D. | N.D. | N.D. |
| **β-3** | THC | 6 | 7.59 | 0.52 | 6.93 | 8.30 |
| | CBD | | 11.96 | 0.84 | 10.79 | 12.89 |
| | THCA | | N.D. | N.D. | N.D. | N.D. |
| | CBDA | | 0.14 | 0.02 | 0.11 | 0.16 |
| | CBN | | 0.31 | 0.06 | 0.25 | 0.40 |
| **β-4** | THC | 27 | 8.04 | 1. 83 | 5.20 | 12.19 |
| | CBD | | 13.05 | 1.78 | 9.85 | 15.58 |
| | THCA | | 0.03 | 0.02 | 0.01 | 0.07 |
| | CBDA | | 0.36 | 0.36 | 0.11 | 1.35 |
| | CBN | | 0.32 | 0.12 | 0.13 | 0.61 |
| **β-5** | THC | 6 | 7.76 | 0.66 | 6.86 | 8.45 |
| | CBD | | 13.15 | 0.94 | 12.04 | 14.64 |
| | THCA | | N.D. | N.D. | N.D. | N.D. |
| | CBDA | | 0.22 | 0.03 | 0.18 | 0.24 |
| | CBN | | 0.31 | 0.06 | 0.24 | 0.40 |

Considering the results obtained, it was concluded that the most efficient method for preparing the oils was the one called β-4. The average concentrations reported in the table, in particular for THC and CBD, allow affirmation that extracting the drug through the method in question was particularly significant. Considering in fact the concentration of the plant drug (both before the start and at the end of the experiments), the oil prepared could contain - at most - quantities of THC between 10.86 mg/ml and 10.28 mg/ml and quantities of CBD between 15.84 mg/ml and 14.46 mg/ml. The oil prepared with the β-4 method contains on average 8.04 mg/ml of THC and 13.05 mg/ml of CBD.

### Pharmaceutical forms for oral use

The capsules prepared with the lipophilic liquid phase prepared with the β-4 method and analyzed with the above method were found to be uniform in terms of content and, therefore, have met the assay required by the Pharmacopoeia. The quantity of lipophilic liquid phase present within each individual dosage unit was in fact less than 10% from the average value.

### Stability test

The stability tests performed for the lipophilic liquid phase carried out with the β-4 method and for the capsules gave excellent results: the variation in the content of decarboxylated active molecules (the quantity of acid forms in the oils was so small that it is not significant) was less than 10% after 180 days of storage in the refrigerator and after 180 days of storage at room temperature.

## Claims

1. A method for extracting delta-9-tetrahydrocannabinol (THC) and cannabidiol (CBD) from *Cannabis* inflorescences, wherein the method comprises the following steps:
(i) triturating the inflorescences of *Cannabis* obtaining a triturated mass;
(ii) preheating the triturated mass in an oven at a temperature between 120 and 145°C, wherein the triturated mass subjected to preheating has a thickness of less than or equal to 5 mm, obtaining a preheated triturated mass;
(iii) mixing the preheated triturated mass with a lipophilic solvent, obtaining a solid-liquid mixture;
(iv) heating the solid-liquid mixture in a water bath with boiling water;
(v) separating a lipophilic liquid phase and a solid phase from the heated solid-liquid mixture,
where the lipophilic liquid phase contains THC and CBD.

2. A method according to claim 1, wherein the lipophilic solvent is selected from virgin olive oil and a pharmaceutically acceptable vegetable oil, preferably virgin olive oil.

3. A method according to any one of the preceding claims, wherein in step (iii), the preheated triturated mass is mixed with the lipophilic solvent in a weight (g)/volume (ml) ratio within the range from 0.1:1 to 0.3:1, preferably equal to 0.2:1.

4. A method according to any one of the preceding claims, wherein separation of the phase (v) envisages pressing and/or filtering the heated solid-liquid mixture.

5. A method according to any one of the preceding claims, wherein the heating step (iv) is carried out for a period of time from 30 to 120 minutes, preferably equal to 60 minutes.

6. A method according to any one of the preceding claims, wherein the preheating step (ii) is carried out at a temperature of 140°C for a period of time from 20 to 45 minutes, preferably equal to 30 minutes.

7. A method according to any one of the preceding claims, wherein at the end of step (iv) the heated solid-liquid mixture is allowed to cool, preferably for a period of time comprised between 10 and 40 minutes.

8. A solid formulation containing THC and CBD, wherein the solid formulation comprises a solid matrix and a lipophilic liquid phase containing THC and CBD, wherein the lipophilic liquid phase containing THC and CBD is obtained by carrying out a method according to any one of claims 1 to 7.

9. A solid formulation according to claim 8, wherein the solid matrix is selected from silica, starch, talc, magnesium stearate or a mixture of the substances in question, preferably silica.

10. A solid formulation according to claim 8 or claim 9, wherein the solid matrix is present in the solid formulation with respect to the lipophilic liquid phase containing THC and CBD in a weight ratio between 1:5 and 1:70, preferably equal to 1:50.

11. A solid formulation according to any one of claims 8 to 10, wherein the solid formulation is encapsulated in a gelatin capsule for pharmaceutical use, or is pressed into a tablet form, optionally coated with a pharmaceutically acceptable coating film.
